# EUROPEAN PATENT APPLICATION

(11) **EP 4 585 161 A1**
(43) Date of publication of application: **16.07.2025**
(21) Application number: 24151560.0
(22) Date of filing: 12.01.2024
(51) Int. Cl.: A61B 6/58, G01R 31/42, G01R 31/56

(54) **LOCALISING POWER SUPPLY DISTURBANCES**

(71) Applicant: Koninklijke Philips N.V., 5656 AG Eindhoven (NL)
(72) Inventor: VOGTMEIER, Gereon, Eindhoven (NL); WEIß, Steffen, Eindhoven (NL); LIPS, Oliver, 5656AG Eindhoven (NL)
(74) Representative: Philips Intellectual Property & Standards

(57) **Abstract**

A monitoring system (100) for localising power supply disturbances in a power supply circuit (110) comprising a power supply (120) and a medical device (130), is provided. The monitoring system (100) comprises an electrical circuit (140) configured to: measure, at an external location (150) with respect to the medical device (130), a characteristic of the power supplied by the power supply (120); measure, at an internal location (160) with respect to the medical device (130), the characteristic of the power supplied by the power supply (120); determine, based on a difference between the measured characteristic at the external location (150), and the measured characteristic at the internal location (160), an origin of a disturbance to the power supplied by the power supply (120) as one of: i) within the medical device (130), and ii) external to the medical device (130); and output an indication of the origin of the disturbance.

## Description

### FIELD OF THE INVENTION

The present disclosure relates to localising power supply disturbances in a power supply circuit that includes a power supply and a medical device. A monitoring system, a computer-implemented method, a computer program product, a service handling system, and a medical device, are disclosed.

### BACKGROUND OF THE INVENTION

Medical devices are typically coupled to a power supply to provide a power supply circuit. For instance, medical imaging systems such as computed tomography "CT" imaging systems, projection X-ray imaging systems, magnetic resonance imaging, "MRI", systems, ultrasound imaging systems, and so forth, are typically coupled to a mains power supply to provide a power supply circuit.

Occasionally, the performance of a medical device can become degraded. Sometimes the origin of the performance degradation is a fault within the medical device. For instance, the X-ray tube within a CT imaging system, or a projection X-ray imaging system can exhibit a fault known as arcing. Arcing can lead to a performance degradation of the imaging system in the form of degraded image quality.

Degradations in the performance of a medical device can also originate from external factors, and these external factors are sometimes confused with faults in the medical device. An example of such an external factor is a power supply disturbance that originates outside of a CT imaging system. Power supply disturbances such as such as transients, fluctuations, interference, and noise, and which originate outside of the CT imaging system, can feed-through into the CT imaging system and likewise give rise to a performance degradation in the form of degraded image quality. The externally-originating power supply disturbance therefore risks being mis-interpreted as a fault within the medical device.

When a performance degradation is detected in a medical device, a service engineer is typically tasked with investigating its origin. The task of finding a fault within a medical device is time-consuming owing to the complexity of medical devices. This task is further complicated by the possibility that, as in the example described above, the origin of the performance degradation is in fact a power supply disturbance that originates outside of the medical device, and is not a fault within the medical device at all.

Thus, there remains a need to support service engineers in determining whether the origin of a performance degradation of a medical device is an external disturbance to the power supplied by the power supply to the medical device, or a fault within the medical device, in order that a service engineer may be efficiently guided to investigate its cause.

### SUMMARY OF THE INVENTION

According to one aspect of the present disclosure, a monitoring system for localising power supply disturbances in a power supply circuit comprising a power supply and a medical device, is provided. The monitoring system comprises an electrical circuit configured to:
measure, at an external location with respect to the medical device, a characteristic of the power supplied by the power supply;
measure, at an internal location with respect to the medical device, the characteristic of the power supplied by the power supply;
determine, based on a difference between the measured characteristic at the external location, and the measured characteristic at the internal location, an origin of a disturbance to the power supplied by the power supply as one of: i) within the medical device, and ii) external to the medical device; and
output an indication of the origin of the disturbance.

In the above monitoring system, a characteristic of the power supplied by the power supply is measured both at an external location with respect to the medical device, and at an internal location with respect to the medical device. The characteristic may be the voltage, the current, or the power, for example. A difference between the measured characteristic at the external location, and the measured characteristic at the internal location, is used to determine the origin of a disturbance to the power supplied by the power supply as one of: i) within the medical device, and ii) external to the medical device.

The inventors have observed that faults within medical devices can give rise to power supply disturbances. Such faults can result in power supply disturbances that are measurable, via the characteristic of the power supplied by the power supply, at the internal location, and which are not measurable, or are at least measurable to a lesser degree, at the external location. The inventors have also observed that power supply disturbances that originate outside of the medical device are measurable, via the characteristic of the power supplied by the power supply, at the external location, and are not measurable, or are at least measurable to a lesser degree, at the internal location. Consequently, the origin of a disturbance to the power supplied by the power supply may be determined as one of: i) within the medical device, and ii) external to the medical device, based on the difference between the measured characteristic at the external location, and the measured characteristic at the internal location.

By providing this information, the monitoring system may be used to efficiently determine whether a performance degradation of a medical device originates from a fault within the medical device, or whether the performance degradation instead originates from outside of the medical device and is due to an external disturbance to the power supplied by the power supply. This information may be used to efficiently guide a service engineer in an investigation of the cause of the power supply disturbance.

Further aspects, features, and advantages of the present disclosure will become apparent from the following description of examples, which is made with reference to the accompanying drawings.

### BRIEF DESCRIPTION OF THE DRAWINGS

Fig. 1 is a schematic diagram illustrating an example of a monitoring system 100 for localising power supply disturbances in a power supply circuit 110 comprising a power supply 120 and a medical device 130, in accordance with some aspects of the present disclosure.
Fig. 2 is a flowchart illustrating an example of a computer-implemented method of localising power supply disturbances, in accordance with some aspects of the present disclosure.
Fig. 3 is a flowchart illustrating an example of a computer-implemented method of handling service requests, in accordance with some aspects of the present disclosure.

### DETAILED DESCRIPTION OF EMBODIMENTS

Examples of the present disclosure are provided with reference to the following description and figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a monitoring system, may be implemented in a computer-implemented method, and in a computer program product, and in a medical imaging system, and in a service handling system, in a corresponding manner.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid-state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray^{™} and DVD.

It is also noted that some operations that are described as being performed by the one or more processors of the systems disclosed herein may be implemented using artificial intelligence techniques. Suitable techniques may include machine learning techniques, deep learning techniques, and neural networks. For instance, one or more neural networks, may be trained in a supervised, or in some cases unsupervised, manner, to implement the operations that are performed by the one or more processors.

As mentioned above, there remains a need to support service engineers in determining whether the origin of a performance degradation of a medical device is an external disturbance to the power supplied by the power supply to the medical device, or a fault within the medical device, in order that a service engineer may be efficiently guided to investigate its cause.

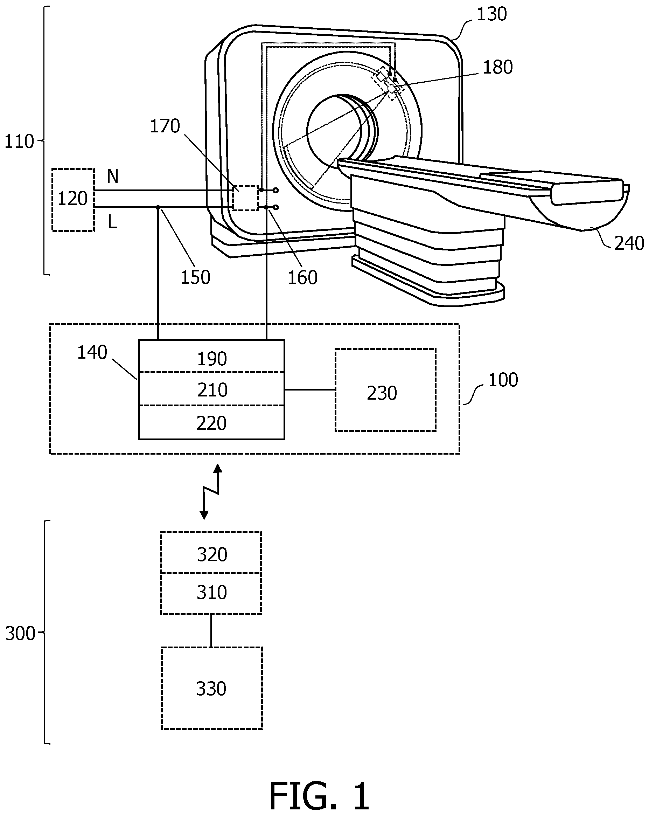

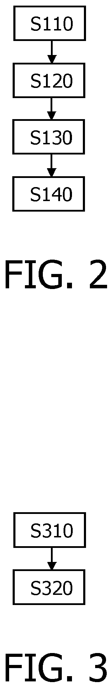

Fig. 1 is a schematic diagram illustrating an example of a monitoring system 100 for localising power supply disturbances in a power supply circuit 110 comprising a power supply 120 and a medical device 130, in accordance with some aspects of the present disclosure. Fig. 2 is a flowchart illustrating an example of a computer-implemented method of localising power supply disturbances, in accordance with some aspects of the present disclosure. It is noted that operations that are described as being performed by the monitoring system 100 illustrated in Fig. 1, may also be performed in the method illustrated in Fig. 2. Likewise, operations that are described in relation to the method described with reference to Fig. 2 may also be performed by the monitoring system 100 illustrated in Fig. 1. With reference to Fig. 1, and Fig. 2, the monitoring system 100 comprises an electrical circuit 140 configured to:
measure S110, at an external location 150 with respect to the medical device 130, a characteristic of the power supplied by the power supply 120;
measure S 120, at an internal location 160 with respect to the medical device 130, the characteristic of the power supplied by the power supply 120;
determine S130, based on a difference between the measured characteristic at the external location 150, and the measured characteristic at the internal location 160, an origin of a disturbance to the power supplied by the power supply 120 as one of: i) within the medical device 130, and ii) external to the medical device 130; and
output S 140 an indication of the origin of the disturbance.

In the above monitoring system, a characteristic of the power supplied by the power supply is measured both at an external location with respect to the medical device, and at an internal location with respect to the medical device. The characteristic may be the voltage, the current, or the power, for example. A difference between the measured characteristic at the external location, and the measured characteristic at the internal location, is used to determine the origin of a disturbance to the power supplied by the power supply as one of: i) within the medical device, and ii) external to the medical device.

The inventors have observed that faults within medical devices can give rise to power supply disturbances. Such faults can result in power supply disturbances that are measurable, via the characteristic of the power supplied by the power supply, at the internal location, and which are not measurable, or are at least measurable to a lesser degree, at the external location. The inventors have also observed that power supply disturbances that originate outside of the medical device are measurable, via the characteristic of the power supplied by the power supply, at the external location, and are not measurable, or are at least measurable to a lesser degree, at the internal location. Consequently, the origin of a disturbance to the power supplied by the power supply may be determined as one of: i) within the medical device, and ii) external to the medical device, based on the difference between the measured characteristic at the external location, and the measured characteristic at the internal location.

By providing this information, the monitoring system may be used to efficiently determine whether a performance degradation of a medical device originates from a fault within the medical device, or whether the performance degradation instead originates from outside of the medical device and is due to an external disturbance to the power supplied by the power supply. This information may be used to efficiently guide a service engineer in an investigation of the cause of the power supply disturbance.

The monitoring system described above may be used to localise power supply disturbances in power supply circuits that are provided by various types of power supplies 120, and various types of medical devices 130. By way of some examples, the power supply 120 may be a so-called "mains" power supply, such as the 220 - 240 Volt, 50 Hz, single or three-phase, mains power supply that is available in Europe and in the UK, and the 240/120 Volt, 60 Hz split-phase, or three-phase, mains power supply that is available in North America. The power supply 120 may alternatively be a generator, or a battery. The medical device 130 may be a medical imaging system, such as a computed tomography "CT" imaging system, or a projection X-ray imaging system, or an MRI imaging system, or a single photon emission computed tomography "SPECT" imaging system, or a positron emission tomography "PET" imaging system, or an ultrasound imaging system, or a intravascular ultrasound "IVUS" imaging system, or an optical coherence tomography "OCT" imaging system, for example. The medical device may alternatively be another type of medical device that serves a different purpose than medical imaging. For instance, the medical device 130 may alternatively be a patient vital signs monitoring device, a respiratory assistance device, an infusion device, and so forth. In the example illustrated in Fig. 1, the power supply circuit 110 comprises a power supply 120 in the form of a mains power supply, and a medical device 130 in the form of a CT imaging system.

In the example illustrated in Fig. 1, the monitoring system 100 is used to determine the origin of a disturbance in a CT imaging system. The CT imaging system includes an X-ray tube. The origin of the disturbance may be an arcing event within the X-ray tube of the CT imaging system. In another example, the monitoring system 100 is used to determine the origin of a disturbance in a projection X-ray imaging system. The projection X-ray imaging system includes an X-ray tube. The origin of the disturbance may be an arcing event within the X-ray tube of the projection X-ray imaging system.

The monitoring system 100 may be used to localise various types of disturbance to the power supplied by the power supply 120. A disturbance is defined herein to be a change to the normal power supplied by the power supply 120. Examples of disturbances include transients, fluctuations, interference, and noise.

The operations that are performed by the electrical circuit 140 are described in more detail below.

Referring initially to the operation S110; in this operation, a characteristic of the power supplied by the power supply 120 is measured at an external location 150 with respect to the medical device 130.

The characteristic of the power supply that is measured in the operation S 110 may be a voltage, or a current, or a power. The characteristic may be measured continuously, or periodically, over time by the monitoring system 100. The characteristic that is measured in the operation S 110 may be measured using various known electrical circuits. For instance, a voltage measurement circuit, or a current measurement circuit, or a power measurement circuit may be used to measure the characteristic in the electrical circuit 140. The use of analogue, and also digital circuits in the electrical circuit 140 is contemplated. The electrical circuit 140 may include, or be coupled to, one or more processors. In the example illustrated in Fig. 1, the electrical circuit includes an analogue-to-digital "ADC" converter 190 to measure the characteristic. The electrical circuit is coupled to one or more processors 210 which may then perform further operations on the measured characteristic, as described below.

The characteristic that is measured in the operation S 110 may be measured by galvanically, or alternatively by non-galvanically, coupling the electrical circuit 140 to the power supply 120. For example, the electrical circuit 140 may be galvanically coupled to the power supply 120 by using a wire that provides electrical contact between the electrical circuit 140 and the power supply 120. This example is illustrated in Fig. 1, wherein the electrical circuit 140 is galvanically coupled to the live terminal, labelled "L", of the mains power supply 120. Alternatively, the electrical circuit 140 may be non-galvanically coupled to the power supply 120. For instance, a coil, or a (stray) capacitance may be used to provide inductive, or capacitive, coupling between the electrical circuit 140 and the power supply 120. Measuring the characteristic using non-galvanic coupling has the advantage of a simpler installation of the monitoring system 100.

The external location 150 may be any location outside of, i.e. external to, the medical device. The external location 150 may be any location outside of a housing of the medical device. In the example of the power supply being a mains power supply, the external location may for instance be a position on a power cable that connects the medical device to the mains power supply, or at a wall plug connection between the mains power supply and the medical device, or at a fusebox connection in the mains power supply.

Referring now to the operation S 120; in this operation the characteristic of the power supplied by the power supply 120 is measured at an internal location 160 with respect to the medical device 130.

The characteristic of the power supplied by the power supply 120 may be measured at the internal location 160 in the operation S120 using the same techniques described above in the operation S110. For example, the ADC 190 illustrated in Fig. 1 may be used to measure the characteristic.

The internal location 160 may be any location within the medical device 130. The internal location 160 may be any location within a housing of the medical device. For instance, in the example illustrated in Fig. 1, the internal location 160 is within the housing of the CT imaging system 130. The internal location 160 may for instance be a position within the housing that is on a cable that connects the medical device 130 to the power supply 120.

Referring now to the operation S 130; in this operation a difference between the measured characteristic at the external location 150, and the measured characteristic at the internal location 160, is used to determine an origin of a disturbance to the power supplied by the power supply 120 as one of: i) within the medical device 130, and ii) external to the medical device 130.

The difference that is evaluated in the operation S130 may for instance be a difference in a magnitude, or a difference in a phase, or a difference in a time (e.g. a delay) or a difference in a signal shape, or a difference in a spectrum, of the measured characteristic.

The difference may be evaluated in various ways in the operation S130. For instance, in the example described above with reference to Fig. 1 and in which the ADC 190 is used to measure the characteristic at the external location 150, and at the internal location 160, a difference may be calculated from the digitised measurements of the characteristics by using the one or more processors 210 to subtract, or to calculate a ratio of, their digitised values that are provided by the ADC. Instead of calculating a difference from the digitised measurements of the characteristic, a difference between the characteristics may be evaluated using their analogue values. For instance, a differential amplifier, or a comparator, may be used to calculate their difference. An output of the differential amplifier, or the comparator, may then be digitised using the ADC, following which the one or more processors 210 may then perform further operations on the measured characteristics, as described below.

The difference that is evaluated in the operation S 130 is then used to determine an origin of a disturbance to the power supplied by the power supply 120 as one of: i) within the medical device 130, and ii) external to the medical device 130.

The difference that is evaluated in the operation S130 may be calculated in various ways. For instance, the difference may be evaluated by subtracting the measured characteristic at the external location 150 from the measured characteristic at the internal location 160, or vice versa, or by calculating a ratio between the measured characteristic at the external location 150 and the measured characteristic at the internal location 160.

In one example, a difference between a magnitude of the measured characteristic at the external location 150, and a magnitude of the measured characteristic at the internal location 160, is used to determine the origin of the disturbance in the operation S 130. The difference between the magnitude of the characteristic at the external location 150, and the magnitude of the measured characteristic at the internal location 160 may be evaluated by subtraction, or by calculating a ratio. In this example, the origin of the disturbance may be determined as the location 150, 160, in which the measured characteristic has the largest magnitude. For instance, an arcing fault that originates within an X-ray tube of the medical device 130 may result in a disturbance in the form of a voltage transient, i.e. a voltage spike, in the power supplied by the power supply 120. The magnitude of the measured voltage transient is expected to be relatively larger at the internal location 160 than at the external location 150 due to the proximity of its origin. By determining the origin of the disturbance as the location in which the measured characteristic has the largest magnitude, the origin of the disturbance is correctly determined as the internal location 160. By contrast, a disturbance that originates outside of the medical device, such as a switching event in another electrical device that is also coupled to the power supply 120, may likewise result in a disturbance in the form of a voltage transient in the power supplied by the power supply 120. In this case, the magnitude of the measured voltage transient is expected to be relatively larger at the external location 150 than at the internal location 160. By determining the origin of the disturbance as the location in which the measured characteristic has the largest magnitude, the origin of the disturbance is in this case correctly determined as the external location 150. The origins of other disturbances, such as noise, and interference may be determined based on differences between the magnitudes of their measured characteristics in a similar manner.

Instead of, or in addition to using the difference between the magnitudes of a measured characteristic to determine the origin of a disturbance, a difference between the phase, or the time, or the signal shape, or the spectrum, of the measured characteristic at the external location 150 and at the internal location 160 may be used to determine the origin of a disturbance. Differences in the phase, or the time, or the signal shape, or the spectrum, of the measured characteristic can also be expected between the external location 150, and the internal location 160, depending on the origin of the disturbance to the power supplied by the power supply. Again, this may be due to the proximity of the origin of the disturbance to the measurement location. For instance, a disturbance may be detected with a time delay, or a phase delay, at the location 150, 160 at which it does not originate, as compared to at its originating location 150, 160. Similarly, a disturbance may be detected with a smoother shape, or with reduced high frequency spectral content at the location 150, 160 at which it does not originate, as compared to at its originating location 150, 160. A difference between the phase, or the time, or the signal shape, of the measured characteristic at the external location 150 and the measured characteristic at the internal location 160 may be evaluated by subtraction. A difference between the spectrum of the measured characteristic at the external location 150 and the spectrum of the measured characteristic at the internal location 160 may be evaluated by calculating a ratio.

Referring now to the operation S 140; in this operation, an indication of the origin of the disturbance is outputted. The indication of the origin of the disturbance may be outputted in various ways. For example, the indication may be outputted to a display device such as to the display 230 illustrated in Fig. 1, or to a virtual/ augmented reality display device, or to a printer, or to a computer-readable storage medium, or to the Internet, or to the Cloud, and so forth. By providing this indication, a service engineer may be efficiently guided to investigate the cause of the disturbance. For instance, if the origin of the disturbance is within the medical device 130, a service engineer may be tasked with investigating the origin of a fault with the medical device 130. By contrast, if the origin of the disturbance is external to the medical device 130, a service engineer may instead investigate potential improvements to the quality of the power supply.
Further examples of the monitoring system 100 are described below.

In one example, the power supply circuit includes at least one electrical component 170, and the medical device 130 is electrically coupled to the power supply 120 via the at least one electrical component 170. The at least one electrical component 170 is configured to filter the power supplied by the power supply 120 to the medical device 130. The external location 150 is at a power supply side of the at least one electrical component 170, and the internal location 160 is at a medical device side of the at least one electrical component 170.

In this example, the at least one electrical component 170 may form part of the power supply 120, or alternatively it may form part of the medical device 130, or part of the monitoring system 100. For instance, it may form part of the electrical circuit 140. The at least one electrical component 170 may be disposed within the medical device 130. Alternatively, at least one electrical component 170 may be disposed outside of the medical device 130. An example in which the at least one electrical component 170 is disposed within the medical device is illustrated in Fig. 1, and wherein the at least one electrical component 170 is disposed within the housing of the CT imaging system 130.

The filtering that is provided by the at least one electrical component 170 in this example has the effect of increasing the difference between the measured characteristic at the external location 150 and the measured the characteristic at the internal location 160. In the operation S 130, this provides a more accurate determination of the origin of a disturbance as i) within the medical device 130, and ii) external to the medical device 130. For instance, the filtering provides a degree of isolation between the measurement of the characteristic at the external location 150 and the measurement of the characteristic at the internal location 160. Consequently, disturbances to the power supply that originate on one side of the at least one electrical component 170 are measured on the originating side of the at least one electrical component 170 with a relatively larger magnitude than at the opposite side of the at least one electrical component 170. In other words, a disturbance originating within the medical device 130 is measured at the internal location 160 with a relatively larger magnitude than at the external location 150. Similarly, disturbances to the power supply that originate outside of the medical device 130 are measured at the external location 150 with a relatively larger magnitude than at the internal location 160.

The filtering that is provided by the at least one electrical component 170 in this example also increases a time difference, a phase difference, a difference in signal shape, and a difference in a spectrum of the measured characteristic between the measured characteristic at the originating side of the at least one electrical component 170 and the measured characteristic at the opposite side of the at least one electrical component 170. In the operation S 130, this likewise provides a more accurate determination of the origin of a disturbance as i) within the medical device 130, and ii) external to the medical device 130.

It is noted that even in the absence of the at least one electrical component 170, differences between the measured characteristic at the external location 150, and the measured characteristic at the internal location 160, still occur due to the proximity of the origin of the disturbance to the measurement location. This is in-part due to the inherent resistance in the path of the power supply circuit 110 between their locations.

In a related example, the at least one electrical component 170 is configured to provide a low pass filter to the power supplied by the power supply 120 to the medical device 130. The low pass filter may attenuate frequencies that are above a mains frequency, i.e. above approximately 50Hz, or above approximately 60 Hz. The low pass filter may attenuate frequencies above approximately 1kHz, or above approximately 10kHz, or above approximately 20kHz, for example. Such a configuration may be provided within the power conditioning circuits of medical devices. For example, medical devices often include a power conditioning circuit that is used to smooth, or to convert, the voltage of the power supply 120 to a voltage that is used within the medical devices. Such power conditioning circuits typically include electrical components such as inductors, capacitors, voltage regulators, a switching power supply, and so forth, and which have the effect of providing a low pass filter to the power supplied by the power supply 120 to the medical device 130. Switching power supplies exploit a common principle wherein a current is switched into a load via one or more non-linear circuit elements, such as diodes, in order to provide a desired output voltage across the load. These likewise provide the effect of a low pass filter to the power supplied by the power supply 120 to the medical device 130.

In one example, the electrical circuit 140 is further configured to:
determine, based on the measured characteristic, a magnitude of the power supply disturbance; and
output an indication of the magnitude of the power supply disturbance.

In this example, the magnitude of the power supply disturbance is indicative of the severity of the disturbance. The outputted indication of the magnitude of the power supply disturbance may therefore be used by a service engineer in determining an urgency with which the disturbance should be investigated. The magnitude of the power supply disturbance may be determined based on factors such as an amplitude, or a root mean square value, or an integrated value, of the measured characteristic, for example. The magnitude of the power supply disturbance may be determined by applying known signal processing techniques to the measured characteristic. The signal processing techniques may be applied by the one or more processors 210, for example.

In another example, the electrical circuit 140 is further configured to:
determine, based on the measured characteristic, a type of the power supply disturbance; and
output an indication of the type of the power supply disturbance.

Examples of types of power supply disturbance that may be determined in this example include power supply disturbance types such as transients, fluctuations, interference, and noise. Such types of power supply disturbances may originate within the medical device 130, or they may originate outside of the medical device.

In this example, the type of the power supply disturbance may be determined by using an algorithm to determine a similarity between the measured characteristic and known samples of different types of interference. The algorithm may be implemented using known signal processing techniques, or using machine learning techniques. Alternatively, the type of the power supply disturbance may be determined by inputting the measured characteristic into a neural network that is trained to predict, from an inputted measured characteristic, a type of power supply disturbance in the measured characteristic. The neural network may be trained to predict the type of power supply disturbance using training data comprising a plurality of measured characteristics comprising a power supply disturbance, and for each measured characteristic, corresponding ground truth data comprising an indication of the type of the power supply disturbance. The training data for this example may be obtained by acquiring temporal measurements of the characteristic. The corresponding ground truth data may be provided by expert analysis of the measurements. Alternatively, the training data may be obtained by simulating disturbances in measured temporal characteristics, the ground truth type of disturbance in this case being known from the simulated disturbances. For instance, simulated disturbances such as noise, interference, and so forth may be superimposed onto measured characteristics. The neural network may be implemented by the one or more processors 210 of the electrical circuit 140 illustrated in Fig. 1.

In a related example, the operation of determining a type of the power supply disturbance includes identifying a component 180 within the medical device 130 in which the disturbance originated. This example may be implemented using the neural network described above, and wherein the training data more specifically represents types of power supply disturbances that are provided by individual components within the medical device. The training data for this example may be obtained from temporal measurements of the characteristic. The ground truth data may be provided by analysing historical service records for the medical device in which the origins of disturbances are recorded. Examples of types of power supply disturbances that may be determined in this example, include transients originating from arcing events in an X-ray tube, transients originating from arcing events in a generator that is configured to supply power to an X-ray tube, interference resulting from the activation of a gantry motor in a CT imaging system, interference resulting from the activation of a collimator in a projection X-ray imaging system, and so forth. An example of a component 180 within a medical device 130 that may be identified as an origin of a disturbance in this example is the X-ray tube 180 of the CT imaging system illustrated in Fig. 1.

In another example, the electrical circuit 140 is further configured to input the measured characteristic at the internal location 160, into an algorithm, and to predict, using the algorithm, and based on the measured characteristic, one or more of:
an expected failure of a component 180 within the medical device 130 and/or one or more maintenance operations for mitigating an expected failure of a component 180 within the medical device 130; and
a remaining useful lifetime of a component 180 within the medical device 130.

Providing this information facilitates a timely repair, or replacement of the component 180. In this example, the characteristic may be measured continuously, or periodically, over time. The algorithm may be implemented by the one or more processors 210 of the electrical circuit 140 illustrated in Fig. 1. The algorithm may be implemented using a model, or using a neural network. Some examples of models that may be used to calculate the replacement time window, or the expected lifetime, are described in a document by Kang, Z., et al., "Remaining Useful Life (RUL) Prediction of Equipment in Production Lines Using Artificial Neural Networks", Sensors 2021, 21(3), 932. A review of such models is also described in a document by Ahmadzadeh, F., et al., "Remaining useful life estimation", Int. J. Syst. Assur. Eng. Manag. 2014, 5, 461-474. These include physics-based models (e.g. physical model, cumulative damage, hazard rate, proportional hazard rate, nonlinear dynamics), experimental-based models, data-driven models (e.g. neural network, support vector machine, Bayesian network, hidden), hybrid models (e.g. statistical models, Fourier transform with neural network, statistical model with neural network, fuzzy logic with neural network, wavelet transform analysis with a statistical model, dynamic wavelet with neural network).

In a related example, the electrical circuit 140 is further configured to generate a service request for repairing and/or replacing the component, or a parts order for the component 180. These operations may be implemented by the one or more processors 210 of the electrical circuit 140 illustrated in Fig. 1. The service request may be transmitted via the first communication unit 220 illustrated in Fig. 1 to the remote service handling system 300 illustrated in Fig. 1. The first communication unit 220 may communicate with the remote service handling system 300 using any form of data communication. For instance, the first communication unit 220 may communicate with the remote service handling system 300 via wireless communication, or via one or more local area networks "LAN"s or wide-area networks "WAN"s. The remote service handling system 300 may be an Internet-based, or a Cloud-based system, for example.

With reference to Fig. 1, it is noted that the monitoring system 100 may also include one or more of: one or more processors 210; an ADC 190; a first communication unit 220; a medical device, such as the CT imaging system 130 illustrated in Fig. 1; the power supply 120; a display, such as the display 230 illustrated in Fig. 1, for displaying the indication of the origin of the disturbance, other outputs generated by the one or more processors 210, and so forth; a patient bed 240; and a user input device (not illustrated in Fig. 1) configured to receive user input in relation to the operations performed by the one or more processors 210, such as a keyboard, a mouse, a touchscreen, and so forth.

In another example, a computer-implemented method of localising power supply disturbances in a power supply circuit 110 comprising a power supply 120 and a medical device 130, is provided. The method comprises:
measuring S110, at an external location 150 with respect to the medical device 130, a characteristic of the power supplied by the power supply 120;
measuring S120, at an internal location 160 with respect to the medical device 130, the characteristic of the power supplied by the power supply 120;
determining S130, based on a difference between the measured characteristic at the external location 150, and the measured characteristic at the internal location 160, an origin of a disturbance to the power supplied by the power supply 120 as one of: i) within the medical device 130, and ii) external to the medical device 130; and
outputting S140 an indication of the origin of the disturbance.

In another example, a computer program product is provided. The computer program product comprises instructions which when executed by one or more processors, cause the one or more processors to carry out a method of localising power supply disturbances in a power supply circuit 110 comprising a power supply 120 and a medical device 130. The method comprises:
measuring S110, at an external location 150 with respect to the medical device 130, a characteristic of the power supplied by the power supply 120;
measuring S120, at an internal location 160 with respect to the medical device 130, the characteristic of the power supplied by the power supply 120;
determining S130, based on a difference between the measured characteristic at the external location 150, and the measured characteristic at the internal location 160, an origin of a disturbance to the power supplied by the power supply 120 as one of: i) within the medical device 130, and ii) external to the medical device 130; and
outputting S140 an indication of the origin of the disturbance.

In another example, a service handling system 300, is provided. The service handling system comprises one or more processors 310 configured to:
receive S310, from the monitoring system 100, input data representing the expected failure of the component 180 and/or the remaining useful lifetime of a component 180 within the medical device 130; and
generate S320, based on the received input data, a service work order for repairing and/or replacing the component 180, or a parts order for the component 180.

This example is illustrated in Fig. 3, which is a flowchart illustrating an example of a computer-implemented method of handling service requests, in accordance with some aspects of the present disclosure. The input data that is received in this example may be in the form of a service request. The input data may be transmitted by the first communication unit 220 illustrated in Fig. 1, and received by the second communication unit 320 of the remote service handling system 300 illustrated in Fig. 1. The service request may be displayed on a display device, such as the display 330 illustrated in Fig. 1. A service engineer may call, or visit a location in which the medical device is located, in response to the service request.

In another example, a medical device 130 that includes the monitoring system 100, is provided. In this example, the monitoring system 100 comprises one or more processors 210. The one or more processors 210 are configured to perform the operation of determining S130 the origin of the disturbance. This operation may be performed as described above. The one or more processors 210 are also configured to at least one of:
record the origin of the disturbance in a log file of the medical device 130;
output, to a display device, a warning message indicative of the origin of the disturbance;
halt, pause, or repeat, a data acquisition operation by the medical device 130, based on the origin of the disturbance;
correct data acquired during a data acquisition operation by the medical device 130, based on the origin of the disturbance;
adjust a mode of operation of the medical device 130, based on the origin of the disturbance;
schedule a service call to investigate the disturbance, based on the origin of the disturbance;
output a recommended change in a configuration of the medical device 130 for reducing an impact of the disturbance;
classify, based on the measured characteristic at the internal location 160, a severity of a disturbance having an origin within the medical device 130; and
measure a quality metric of data acquired during a data acquisition operation by the medical device 130; identify a degradation in the quality metric coincident with a time of the disturbance; and output an indication of a cause of the degradation in the quality metric as one of: i) within the medical device 130, and ii) external to the medical device 130, based on the origin of the disturbance.

The record of the origin of the disturbance in a log file of the medical device 130 is useful in diagnosing reported performance issues. For instance, if the log file indicates a pattern of disturbances originating outside of the medical device, a response to a reported performance issue may be to recommend an upgrade to the power supply 120 that supplies power to the medical device, rather than to dispatch a service engineer to investigate a fault with the medical device 130.

The outputting of a warning message indicative of the origin of the disturbance may alert an operator of the medical device to the risk of an associated degradation in the performance of the medical device. For instance, the warning message may alert a user to the need to carefully inspect a medical image generated by the medical device for image artifacts. The detection of image artifacts at this stage in the workflow facilitates the acquisition of a re-take image without the interruption to workflow associated with recalling a patient for a re-take image at a later date.

The halting, pausing, or repeating, a data acquisition operation by the medical device 130 may be performed in various situations. For instance if the origin of the disturbance is external to the medical device, imaging might be continued. By contrast, if the origin of the disturbance is within the medical device, it might be desirable to halt, pause, or repeat, a data acquisition operation. These operations may be performed in order to maintain a desired level of quality in the acquired data.

The correction of data acquired during a data acquisition operation by the medical device 130 may be performed in various situations. For instance, the origin of the disturbance is an arcing event in an X-ray tube of an X-ray or CT imaging system, multiple image frames may be affected. In this situation, the data for the affected image frames may be corrected by interpolating the affected data using the data from one or more previously-acquired image frames.

The adjusting of a mode of operation of the medical device 130, based on the origin of the disturbance may be performed in various situations. For instance, if the origin of the disturbance is an arcing event in an X-ray tube of an X-ray or CT imaging system, it may be beneficial to adjust the mode of operation of the medical device 130 by switching the X-ray tube to a lower energy "kV" setting. This reduces the risk of further arcing. Similarly, if the origin of the disturbance is an arcing event in an X-ray tube of an X-ray or CT imaging system, it may be beneficial to adjust the mode of operation of the medical device 130 by performing a "conditioning procedure" on the X-ray tube. A conditioning procedure involves the controlled adjustment of the kV setting, and the beam current of the X-ray tube in stages. A conditioning procedure has the effect of removing residual gasses that have been released from the internal surfaces of the X-ray tube and consequently reduces the risk of further arcing.

The scheduling of a service call to investigate the disturbance, based on the origin of the disturbance may be performed in various situations. For instance, a service call might be scheduled if the origin of the disturbance is within the medical device. By contrast, of the origin of the disturbance is outside of the medical device, a service call might not be scheduled.
The outputting of a recommended change in a configuration of the medical device 130 for reducing an impact of the disturbance may be performed in various situations. For instance, if the origin of the disturbance is an arcing event in an X-ray tube of an X-ray or CT imaging system, it may be desirable to switch the X-ray tube to a lower energy "kV" setting in order to reduce the risk of further arcing.

The classification of a severity of a disturbance having an origin within the medical device 130 may be performed in various situations. For instance, if a magnitude of the measured characteristic is below a threshold value, the severity may be classified as negligible. If a magnitude of the measured characteristic exceeds the threshold value, the severity may be classified as significant and also correctable. If a magnitude of the measured characteristic exceeds a relatively higher threshold value, the severity may be classified as severe and also un-correctable.

The outputting of an indication of a cause of the degradation in the quality metric as one of: i) within the medical device 130, and ii) external to the medical device 130, based on the origin of the disturbance may be performed in various situations, such as to provide advice to an operator. In this example, a quality metric of data acquired during a data acquisition operation by the medical device 130 may be measured by using e.g. an algorithm, or a neural network to measure noise, or image artifacts, in medical images generated by the medical device. A degradation in the quality metric coincident with a time of the disturbance may be determined by periodically evaluating the quality metric and determining whether any disturbances have occurred during a time window over which the data is acquired. The cause of the degradation in the quality metric may then be outputted, e.g. to the display device 230 illustrated in Fig. 1, in order to inform an operator of the degraded image quality. Indicating the cause of the disturbance in accordance with this example enables the operator to determine a course of action for resolving the degradation in image quality.

In a related example, the monitoring system 100 that is included within the medical device 130 is configured to communicate the indication of the magnitude of the power supply disturbance to the one or more processors 210. In this example, the one or more processors 210 are configured to perform the at least one of:
recording the origin of the disturbance;
outputting a warning message;
halting, pausing, or repeating, a data acquisition operation;
correcting data acquired during a data acquisition operation,
adjusting a mode of operation of the medical device 130;
scheduling a service call; and
recommend a change in a configuration of the medical device 130;
based further on the magnitude of the power supply disturbance.

Thus in this example, the magnitude of the power supply disturbance is used to determine the subsequent course of action. In this example, the course of action might, for example, only be pursued if the magnitude of the power supply disturbance is above a threshold value, or within a predefined range.

The above examples are to be understood as illustrative of the present disclosure, and not restrictive. Further examples are also contemplated. For instance, the examples described in relation to the monitoring system, may also be provided by the computer-implemented method, or by the computer program product, or by the computer-readable storage medium, in a corresponding manner. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. A monitoring system (100) for localising power supply disturbances in a power supply circuit (110) comprising a power supply (120) and a medical device (130), the monitoring system (100) comprising an electrical circuit (140) configured to:
measure (S110), at an external location (150) with respect to the medical device (130), a characteristic of the power supplied by the power supply (120);
measure (S120), at an internal location (160) with respect to the medical device (130), the characteristic of the power supplied by the power supply (120);
determine (S130), based on a difference between the measured characteristic at the external location (150), and the measured characteristic at the internal location (160), an origin of a disturbance to the power supplied by the power supply (120) as one of: i) within the medical device (130), and ii) external to the medical device (130); and
output (S140) an indication of the origin of the disturbance.

2. The monitoring system according to claim 1, wherein the power supply circuit further comprises at least one electrical component (170);
wherein the medical device (130) is electrically coupled to the power supply (120) via the at least one electrical component (170);
wherein the at least one electrical component (170) is configured to filter the power supplied by the power supply (120) to the medical device (130); and
wherein the external location (150) is at a power supply side of the at least one electrical component (170), and the internal location (160) is at a medical device side of the at least one electrical component (170).

3. The monitoring system according to claim 2, wherein the at least one electrical component (170) is configured to provide a low pass filter to the power supplied by the power supply (120) to the medical device (130).

4. The monitoring system according to any previous claim, wherein the characteristic comprises a voltage, or a current, or a power.

5. The monitoring system according to any previous claim, wherein the difference comprises a difference in a magnitude, or a phase, or a time, or a signal shape, or a spectrum, of the measured characteristic.

6. The monitoring system according to any previous claim, wherein the electrical circuit (140) is further configured to:
determine, based on the measured characteristic, a magnitude of the power supply disturbance; and
output an indication of the magnitude of the power supply disturbance.

7. The monitoring system according to any previous claim, wherein the electrical circuit (140) is further configured to:
determine, based on the measured characteristic, a type of the power supply disturbance; and
output an indication of the type of the power supply disturbance.

8. The monitoring system according to claim 7, wherein the determining a type of the power supply disturbance further comprises identifying a component (180) within the medical device (130) in which the disturbance originated.

9. The monitoring system according to any previous claim, wherein the electrical circuit (140) is further configured to input the measured characteristic at the internal location (160), into an algorithm, and to predict, using the algorithm, and based on the measured characteristic, one or more of:
an expected failure of a component (180) within the medical device (130) and/or one or more maintenance operations for mitigating an expected failure of a component (180) within the medical device (130); and
a remaining useful lifetime of a component (180) within the medical device (130).

10. The monitoring system according to any previous claim, wherein the medical device (130) is a computed tomography imaging system comprising an X-ray tube, or a projection X-ray imaging system comprising an X-ray tube; and
wherein the origin of the disturbance within the medical device (130) is an arcing event within the X-ray tube of the computed tomography imaging system, or the projection X-ray imaging system, respectively.

11. The monitoring system according to any previous claim, wherein the disturbance comprises a transient, or a fluctuation, or interference, or noise.

12. A computer-implemented method of localising power supply disturbances in a power supply circuit (110) comprising a power supply (120) and a medical device (130), the method comprising:
measuring (S110), at an external location (150) with respect to the medical device (130), a characteristic of the power supplied by the power supply (120);
measuring (S120), at an internal location (160) with respect to the medical device (130), the characteristic of the power supplied by the power supply (120);
determining (S 130), based on a difference between the measured characteristic at the external location (150), and the measured characteristic at the internal location (160), an origin of a disturbance to the power supplied by the power supply (120) as one of: i) within the medical device (130), and ii) external to the medical device (130); and
outputting (S140) an indication of the origin of the disturbance.

13. A service handling system (300), the service handling system comprising one or more processors (310) configured to:
receive (S310), from the monitoring system according to claim 9, input data representing the expected failure of the component (180) and/or the remaining useful lifetime of a component (180) within the medical device (130); and
generate (S320), based on the received input data, a service work order for repairing and/or replacing the component (180), or a parts order for the component (180).

14. A medical device (130) comprising:
the monitoring system (100) according to any previous claim; and
wherein the monitoring system (100) comprises one or more processors (210); wherein the one or more processors (210) are configured to determine (S130) the origin of the disturbance; and
wherein the one or more processors (210) are further configured to at least one of:
record the origin of the disturbance in a log file of the medical device (130);
output, to a display device, a warning message indicative of the origin of the disturbance;
halt, pause, or repeat, a data acquisition operation by the medical device (130), based on the origin of the disturbance;
correct data acquired during a data acquisition operation by the medical device (130), based on the origin of the disturbance;
adjust a mode of operation of the medical device (130), based on the origin of the disturbance;
schedule a service call to investigate the disturbance, based on the origin of the disturbance;
output a recommended change in a configuration of the medical device (130) for reducing an impact of the disturbance;
classify, based on the measured characteristic at the internal location (160), a severity of a disturbance having an origin within the medical device (130); and
measure a quality metric of data acquired during a data acquisition operation by the medical device (130); identify a degradation in the quality metric coincident with a time of the disturbance; and output an indication of a cause of the degradation in the quality metric as one of: i) within the medical device (130), and ii) external to the medical device (130), based on the origin of the disturbance.

15. The medical device according to claim 14 when dependent on claim 6, wherein the monitoring system is further configured to communicate the indication of the magnitude of the power supply disturbance to the one or more processors (210);
wherein the one or more processors (210) are configured to perform the at least one of:
recording the origin of the disturbance;
outputting a warning message;
halting, pausing, or repeating, a data acquisition operation;
correcting data acquired during a data acquisition operation,
adjusting a mode of operation of the medical device (130);
scheduling a service call; and
recommend a change in a configuration of the medical device (130); based further on the magnitude of the power supply disturbance.
